# EUROPÄISCHE PATENTSCHRIFT

(11) **EP 0 758 885 B1**
(45) Veröffentlichungstag und Bekanntmachung des Hinweises auf die Patenterteilung: **06.12.2000**
(21) Anmeldenummer: 95920804.2
(22) Anmeldetag: 08.05.1995
(51) Int. Cl.: A61K 9/70

(54) **HYDROPHILE HAFTSCHMELZKLEBER**
HYDROPHILIC, HOT-MELT-TYPE PRESSURE-SENSITIVE ADHESIVE
COLLE HYDROPHILE A FUSION SENSIBLE A LA PRESSION

(30) Priorität: 13.05.1994 DE 4416927
(43) Veröffentlichungstag der Anmeldung: 26.02.1997
(73) Patentinhaber: LTS LOHMANN Therapie-Systeme GmbH, 56567 Neuwied (DE)
(72) Erfinder: HOFFMANN, Hans-Rainer, 56566 Neuwied (DE); ROREGER, Michael, 56566 Neuwied (DE)
(74) Vertreter: Flaccus, Rolf-Dieter, Dr.
(86) Internationale Anmeldenummer: EP9501724
(87) Internationale Veröffentlichungsnummer: WO9531188

(56) Entgegenhaltungen:
- EP-A- 0 355 536
- EP-A- 0 460 588
- EP-A- 0 507 160
- WO-A-87/02870
- DE-C- 4 230 588
- FR-A- 2 612 785
- PATENT ABSTRACTS OF JAPAN vol. 16 no. 419,3.September 1992 & JP,A,04 145019 (DAIYA SEIYAKU KK) 19.Mai 1992, & DATABASE WPI Section Ch, Week 9226 Derwent Publications Ltd., London, GB; Class A12, AN 92-214238 & JP,A,04 145 019 (DAIYA SEIYAKU KK) , 19.Mai 1992
- PATENT ABSTRACTS OF JAPAN vol. 17 no. 513,16.September 1993 & JP,A,05 139961 (NITTO DENKO CORP.) 8.Juni 1993, & DATABASE WPI Section Ch, Week 9327 Derwent Publications Ltd., London, GB; Class A04, AN 93-216650 & JP,A,05 139 961 (NITTO DENKO CORP.) , 8.Juni 1993

## Beschreibung

Die vorliegende Erfindung betrifft selbstklebende Produkte zur Anwendung auf intakter Haut mit einem Haftkleber zur Abgabe von Stoffen an bzw. durch die Haut, die im Haftkleber gleichmäßig oder ungleichmäßig verteilt sind, sowie deren Herstellung und Verwendung.

Typische Repräsentanten solcher Produkte sind z.B. medizinische Pflaster zur gesteuerten dermalen oder transdermalen Abgabe vom Stoffen sowie kosmetische Pflaster zur Abgabe von Aktivsubstanzen oder Duftstoffen, selbstklebende Produkte zur Anwendung auf geschädigter Haut, wie z.B. Pflaster, Wundauflagen oder Operationsfolien zur Abgabe von Stoffen an Wunden.

Überwiegend werden haftklebende Schichten, die pharmazeutische Wirkstoffe oder andere Aktivsubstanzen enthalten, durch Aufbringen einer Lösung oder Dispersion mit anschließender Verdampfung des Löse- oder Dispersionsmittels gebildet.

Die Verwendung von Löse- oder Dispersionmitteln bei der Herstellung von Kleberschichten für Produkte der genannten Art hat sowohl in ökonomischer Hinsicht, bedingt durch teure Trocknungsstrecken, Absauganlagen, Wiedergewinnungs- und Entsorgungseinrichtungen, als auch in ökologischer Hinsicht, bedingt durch die Umweltbelastung durch Löse- und Dispersionsmittel sowie deren Abbauprodukte, eine Reihe von Nachteilen.

Kleberschichten, die aus einer Schmelze der Formulierungsbestandteile hergestellt werden, haben demgegenüber in ökonomischer wie ökologischer Sicht Vorteile, weisen aber den Nachteil auf, daß die zum Aufschmelzen der üblicherweise verwendeten Haftschmelzkleberformulierungen notwendigen Temperaturen so hoch sind, daß die einzuarbeitenden, pharmazeutischen Wirkstoffe oder anderen Aktivsubstanzen zumindest teilweise zersetzt, verändert oder verdampft werden.

Darüber hinaus weisen die üblichen Haftschmelzkleberformulierungen, wie auch löse- oder dispersionsmittelgebundene Kleberformulierungen, insbesondere bei Anwendung am Menschen weitere Nachteile auf, die in der Regel während der Anwendung oder nach Entfernen der Vorrichtung in Form von Hautreaktionen und schmerzhaften Rötungen sichtbar werden.

Diese Hautreizungen können bei aggressiv klebenden Produkten bzw. Anordnungen auf eine Verletzung der Horn-schicht beim Abziehen der klebenden Anordnung von der Haut zurückzuführen sein. Am häufigsten hängen Reaktionen der Haut allerdings damit zusammen, daß die Basisbestandteile von Kleberformulierungen lipophiler Natur sind, was insbesondere bei Haftschmelzklebern die Regel ist. Diese Kleber wirken auf der Haut wie okklusive Filme oder Folien, die die Hautatmung und die Evaporation von Wasser und somit die Mechanismen der Thermoregulierung am Anwendungsort vollständig unterbinden. Da das von der Haut abgegebene Wasser nicht verdunsten kann, bilden sich zwischen Kleberschicht und Haut dünne Wasserfilme und Wasserinseln, die ein optimales Millieu für Wachstum und Vermehrung von Hautkeimen darstellen. Ab einer bestimmten Populationsgröße zerfallen dann pro Zeiteinheit so viele Hautkeime, daß die Konzentration an toxischen Abbau- und Zerfallsprodukten stark ansteigt, was wiederum zu massiven Hautreaktionen führt.

Diesen Phänomenen hat man z.B. bei der Haftkleberformulierung für selbstklebende Wundauflagen versucht dadurch zu begegnen, daß in Haftschmelzklebern sogenannte Hydrokolloidpartikel eingearbeitet werden, d.h. hydrophile Substanzen, insbesondere Polymere, die Wasser absorbieren und von der Hautoberfläche abführen.

Aber auch mit derartigen Formulierungen kann ein anderer gravierender Nachteil von üblichen Haftschmelzklebern nicht vermieden werden. Denn neben den aufgezeigten eher physikalischen Ursachen, die zu Hautreizungen führen, sind es vor alle allergische Hautreaktionen, die Probleme bereiten und die in der Regel auf die chemischen Eigenschaften der verwendeten lipophilen Klebharze zurückzuführen sind. Für dieses Problem ist bislang keine befriedigende Lösung gefunden worden, da, wenn ein sicheres Haften auf der Haut erforderlich ist, auf diese Klebharze nicht verzichtet werden kann.

Haftschmelzkleberformulierungen zur Abgabe von Stoffen nach dem Stand der Technik weisen aufgrund ihrer lipophilen Natur einen weiteren Nachteil auf, da sie, was die gesteuerte Abgabe von Stoffen anbetrifft, nur für lipophile Stoffe geeignet sind. Für hydrophile Stoffe können die Voraussetzungen für die Steuerung der Abgabe bezüglich der thermodynamischen Aktivität in dem Haftschmelzkleber kaum geschaffen werden. Damit die Stoffe innerhalb einer Kleberformulierung diffundieren, an die Grenzfläche gelangen und abgegeben werden können, müssen diese Stoffe zumindest teilweise in der Kleberformulierung gelöst sein, was in der Regel bei Kombination hydrophiler Stoffe und lipophiler Haftschmelzkleber nicht gelingt.

Aufgabe de vorliegenden Erfindung war es daher, eine Vorrichtung zur Abgabe von Stoffen aus Haftschmelzklebern zu finden, die einen Haftschmelzkleber umfaßt, der bei Raumtemperatur nach leichtem Andrücken sicher auf der gewünschten Oberfläche haftet, der nach Entfernen keine Rückstände auf der Oberfläche hinterläßt, der Hautfeuchtigkeit absorbiert, der nichtallergene Klebharze enthält und der die Einarbeitung hydrophiler Stoffe in gelöster Form ermöglicht.

Die Lösung der Aufgabe gelingt bei einem selbstklebenden Produkt der eingangs genannten Art dadurch, daß der Haftkleber ein durch Kombination von wasserlöslichen oder zumindest wasserquellbaren Polymeren mit wasserlöslichen, schmelzbaren Klebharzen gebildeter druckempfindlicher hydrophiler Haftschmelzkleber ist.

Überraschenderweise wurde gefunden, daß es möglich ist, durch die Kombination von wasserlöslichen oder zumindest wasserquellbaren Polymeren mit wasserlöslichen, schmelzbaren Klebharzen unter Zusatz der abzugebenden Stoffe druckempfindliche Haftschmelzkleber herzustellen, die den genannten Anforderungen entsprechen, wobei wasserlösliche, schmelzbare, an sich niedrigviskose und fädenziehende klebrige Substanzen in Kombination mit hydrophilen Polymeren zusammenhängende Schichten von ausgezeichneter Kohäsion bilden, die zudem Klebkräfte aufweisen, welche denen herkömmlicherr Kleber durchaus vergleichbar sind.

Aus der EP-A-355 536 sind zwar wirkstoffhaltige Filme mit wasserlöslichen Polymeren bekannt, jedoch handelt es sich hier um flexible hydrophile Gelfilme, die aus anionenaktiven und kationenaktiven Polymeren zusammen mit Feuchthaltemittel und Wasser gebildet sind, wobei durch die Kombination der unterschiedlich geladenen Polymeren die mechanische Festigkeit des Gelfilms begründet wird, dessen Flexibilität aus seinem Gehalt an Feuchthaltemittel und Restwasser resultiert.

Diese Produkte werden nicht aus der Schmelze verarbeitet oder gewonnen, sondern auf sehr interessante Weise durch Anwendung von Lösungsmittel und flüchtigen Zusätzen beim Vermischen der gegensätzlich geladenen Polymeren, wodurch deren Verklumpen vermieden werden kann.

Druckempfindliche Haftschmelzkleber im Sinne dieser Erfindung sind Haftschmelzkleber, mit Hilfe derer bei Raumtemperatur unter geringem Druck beschichtete Vorrichtungen mit Oberflächen zuverlässig verbunden werden können, und die eine Kohäsion aufweisen, die ein rückstandsfreies Entfernen der kleberbeschichteten Vorrichtung von der Anwenderoberfläche durch einfaches Abziehen ermöglicht. Haftschmelzkleber der im Hauptanspruch genannten Art werden, damit es nicht zu unerwünschten Veränderungen der abzugebenden Stoffe oder anderer Formulierungsbestandteile kommt, vorzugsweise bei Temperaturen zwischen 40 und 150 °C und besonders bevorzugt bei Temperaturen zwischen 80 und 120 °C in geschmolzenem Zustand verarbeitet und erstarren unter Aufbau adhäsiver und kohäsiver Kräfte.

Haftschmelzkleber der im Hauptanspruch genannten Art unterscheiden sich deutlich von hydrophilen, wasserlöslichen Klebern, wie sie aus der Herstellung von sogenannten Gummierungsflächen wie z.B. Briefumschläge oder Etiketten bekannt sind. Diese Kleber, die nach Aufschmelzen, Verarbeiten und Erstarren zwar kohäsive aber keinerlei adhäsive Kräfte aufbauen, werden erst durch Befeuchten mit Wasser oder Speichel aktiviert und dann im feuchten Zustand mit der Applikationsfläche in Kontakt gebracht. Nach dem Trocknen des Klebers bilden sich Haftkräfte aus, die die betreffenden Oberflächen, z.B. Papieretiketten mit Glasflächen, fest miteinander verbinden. Diese Haftkräfte zwischen Vorrichtung und Oberfläche können, im Gegensatz zu mittels druckempfindlicher Haftschmelzkleber verbundener Oberlfächen, nicht durch einfaches Abziehen der kleberbeschichteten Vorrichtung von der Anwenderoberfläche aufgehoben weren, sondern in diesen Fällen kann das Aufheben der Haftkräfte nur durch erneutes Lösen oder Anlösen des Klebers erfolgen, z.B. durch Befeuchtung mit Wasser, durch Ablösen im Wasserbad oder über Wasserdampf.

Das Hauptproblem bei der Formulierung von Haftschmelzklebern gemäß der vorliegenden Erfindung bestand darin, Substanzen zu finden, die wasserlöslich und zudem schmelzbar sind, die bei Raumtemperatur adhäsive Eigenschaften besitzen und von denen bekannt ist, daß sie auch bei langer Applikation auf menschlicher Haut keine Hautreizungen oder allergische Reaktionen verursachen. Derartige Substanzen sind selten und bislang vornehmlich unter den Naturstoffen zu finden. Derartig klebrignachende Substanzen, die in Anlehnung an die bestehende Terminologie als Klebharze bezeichnet werden, sind vorzugsweise Pantothenylalkohol, Honig, niedermolekulare Zucker wie z.B. Saccharose, Glucose und Fructose, oder Derivate niedermolekularer Zucker, wie beispielsweise Saccharoseacetatisobutyrat, sowie deren Kombinationen.

Als wasserlösliche oder wasserquellbare Polymere im Sinne der vorliegenden Erfindung kommen alle hydrophilen natürlichen, semisynthetischen oder synthetischen Polymere in Betracht, die bei den verwendeten Temperaturen schmelzen oder sich zumindest teilweise in trockener oder vorgequollener Form in den eingesetzten Klebharzschmelzen lösen. Dazu gehören vorzugsweise Gelatine, pflanzliche Polysaccharide wie Alginate, Pektine, Carrageenane oder Xanthan, Cellulosederivate wie Methylcellulose, Hydroxipropylcellulose, Hydroxiethylcellulose, Hydroxipropylmethylcellulose oder Natriumcarboximethylcellulose, Stärke und Stärkederivate, Galaktomannan und Galaktomannanderivate, Polyvinylalkohol, Polyvinylpyrrolidon, Vinylpyrrolidon-Vinylacetat-Mischpolymerisate, Polyethylengylcole und Polypropylenglycole.

Unter der Bezeichnung "Stoffe" im Zusammenhang mit der vorliegenden Erfindung werden chemische Elemente, organische und anorganische Verbindungen verstanden, die aus den sie enthaltenden Bestandteilen einer gattungsgemäßen Vorrichtung herauswandern können und dadurch einen angestrebten Effekt hervorrufen. Unter den Anwendungsgebieten der erfindungsgemäßen Vorrichtung ist die Human- und Tiermedizin sowie die Anwendung an Pflanzen von besonderer Bedeutung, wobei hier eine Ausgestaltung der Erfindung in Pflasterform besonders bevorzugt ist.

Die erfindungsgemäße Vorrichtung zur Abgabe von Stoffen aus Haftschmelzklebern findet beispielsweise Anwendung auf der intakten Haut oder auf Wunden. Die abzugebenden Stoffe dienen in diesen Fällen vorzugsweise der dermalen Behandlung lokaler Hauterkrankungen, der intra- und transdermalen Behandlung von Erkrankungen, der Wundbehandlung oder der Pflege der Haut in kosmetischen Zubereitungen. Zur dermalen Behandlung lokaler Hauterkrankungen werden Lokalanästhetika, Lokalantibiotika, Antiseptika, Antimykotika, Antihistaminika, und juckreizstillende Arzneistoffe, Keratolytika und ätzende Arzneistoffe, Virustatika, Antiskabiesmittel, Steroide, sowie verschiedene Substanzen zur Behandlung von Akne, Psoriasis oder Lichtdermatosen verwendet. Zu den Wirkstoffen, die intradermal appliziert werden, gehören z.B. steroidale und nichtsteroidale Antirheumatika, Lokalanästhetika, durchblutungsfördernde Substanzen oder Vasoprotektoren und -konstriktoren zur Behandlung von Gefäßerkrankungen sowie Wirkstoffe zur Beeinflussung von Vorgängen im Unterhautfettgewebe. Zu den transdermal applizierten Wirkstoffen gehören z.B. Neuroleptika, Antidepressiva, Tranquillantien, Hypnotika, Psychostimulantien, Analgetika, Cytostatika, Hormone, Muskelrelaxantien, Antiparkinsonmittel, Ganglienblocker, Sympatomimetika, Alpha-Sympatholytika, Beta-Sympatholytika, Antisympathotonika, Antidiabetika, Koronartherapeutika, Antihypertonika, Antiasthmatika, Diuretika oder Wirkstoffe zur Gewichtsreduzierung.

Zur Wundbehandlung werden blutstillende Wirkstoffe, beispielsweise Kollagen, wundreinigende Stoffe, wie beispielsweise Enzyme, Antiseptika, Desinfizientia und Antibiotika, schmerzstillende Mittel und anästhesierende Wirkstoffe sowie wundheilungsfördernde Wirkstoffe zur Anregung der Granulation, zur Induzierung der Gefäßbildung oder zur Förderung der Epithelisierung eingesetzt.

In einer bevorzugten Ausführungsform zur transdermalen Anwendung enthält der hydrophile Haftschmelzkleber Acetylsalicylsäure, vorzugsweise für die antithrombotische Therapie, d.h. zur Verhindung eines erstmaligen Herzinfarktes, zur Verhinderung eines Reinfarktes, zur Behandlung von instabiler Angina Pectoris, zur Thromboseprophylaxe nach Einsatz von Gefäßprothesen bzw. künstlichen Herzklappen, zur Thromboseprophylaxe der peripheren arteriellen Gefäße sowie zur Thromboseprophylaxe cerebraler Mangeldurchblutungen.

In einer anderen bevorzugten Ausführungsform zur transdermalen Anwendung enthält der hydrophile Haftschmelzkleber ein Steroidhormon, vorzugsweise Estradiol, das bei transdermaler Applikation zur Hormonsubstitution in der Postmenopause oder zur Behandlung der Osteoporose eingesetzt wird. Andererseits kann eine Vorrichtung zur Abgabe von Estradiol an hydrophilen Haftschmelzklebern aber auch auf chronischen Wunden beispielsweise Ulcera Cruris, zur Wundbehandlung eingesetzt werden.

In einer bevorzugten Auführungsform zur intradermalen Applikation von Wirketoffen enthält der hydrophile Haftschmelzkleber ein eutektisches Gemisch der Lokalanästhetika Lidocain und Prilocain. Mittels dieser Wirkstoffmischung kann sowohl bei der intradermalen Analgesie vor beispielsweise Venenpunktionen als auch bei der Behandlung der rheumatoiden Arthritis eine Wirkung erzielt werden, die mit den üblicherweise eingesetzten Wirkstoffen und Wirkstoffkombinationen aus der Gruppe der topischen Anästhetika nicht erzielt werden kann.

In einer weiteren bevorzugten Ausführungsform der erfindungsgemäßen Vorrichtung enthält der hydrophile Haftschmelzkleber pflanzliche Zubereitungen wie beispielsweise Extrakte oder Tinkturen. Diese können verwendet werden zur Behandlung lokaler Hauterkrankungen, wie beispielsweise Eichenrindenextrakt, Walnußextrakt, Arnikablütentinktur, Hamamelisrindenextrakt, Spitzwegerichextrakt, Stiefmütterchenextrakt, Thymian- oder Salbeiextrakt, zur Behandlung von geschädigter und verletzter Haut, wie beispielsweise Johanniskrauttinktur, Sonnenhutkrauttinktur, Kamillenblütenextrakt oder Ringelblumenblütentinktur, sowie zur Pflege von strapazierter und geschädigter Haut, wie beispielsweise Birkenblätterextrakt, Brennesselextrakt, Huflattichextrakt, Beinwelltinktur, Schachtelhalmextrakt oder Aloe Vera Extrakt. Pflanzliche Zubereitungen können aus hydrophilen Haftschmelzkbebern aber auch abgegeben werden zur intradermalen Behandlung von Erkrankungen, wie beispielsweise Roßkastanien- und Mäusedornestrakte bei Venenleiden, oder Arnika-, Ringelblumen- und Capsicumextrakte und -tinkturen bei Prellungen, Verstauchungen oder Blutergüssen. Pflanzlidie Zubereitungen in erfindungsgemäßen hydrophilen Haftschmelzklebern können aber auch in der transdermalen Therapie eingesetzt werden, so beispielsweise Ginsengextrakt bei Altersbeschwerden, Baldriantinktur, Melissen- und Hopfenextrakt zur Beruhigung bei Überreizung, Schlafstörungen und Streß, Cola- und Teeextrakte zur Erzielung einer anregenden Wirkung oder Weißdornextrakt zur Kreislaufstabilisierung.

In besonderen Fällen, in denen die Formulierungsbestandteile des hydrophilen Haftschmelzklebers selbst zur Extraktion von Inhaltsstoffen aus Pflanzenteilen fähig sind, kann der hydrophile Haftschmelzklebr auch zerkleinerte Pflanzenteile enthalten. In einer bevorzugten Ausführungsform enthält der hydrophile Haftschmelzkleber der erfindungsgemäßen Vorrichtung Tabakpulver. Eine derartige Vorrichtung kann von Rauchern verwendet werden als Alternative zu Tabak, Zigaretten und ähnlichen Rauchwaren. Dieser Ersatz für beispielsweise Zigaretten bietet den Vorteil, daß zum einen der Raucher selbst die bei der Verbrennung von Tabak entstehenden schädlichen Stoffe nicht einatmet, ohne dabei auf die gewünschten Effekte des Tabakgenusses verzichten zu müssen, und daß zum anderen Nichtraucher den Gefahren des Passivrauchens nicht ausgesetzt werden.

Die erfindungsgemäße Vorrichtung zur Abgabe von Stoffen aus hydrophilen Haftschmelzklebern kann aber auch auf Pflanzen angewandt werden. So können beispielsweise oberflächliche, lokal begrenzte Pflanzenkrankheiten mit Wirkstoffen behandelt werden, die aus dem hydrophilen Haftschmelzkleber an die Pflanzenoberfläche abgegeben werden. Ebenso können Wirkstoffe mit systemischer Wirksamkeit, die dem Schutz der Pflanze vor Krankheitserregern und Schädlingen oder der Behandlung von Pflanzen nach erfolgter Infektion und bereits vorhandenem Befall, der Wachstumregulierung oder der Ernährung der Pflanze dienen, aus dem hydrophilen Haftschmelzkleber an die Pflanzenoberfläche, wie beispielsweise die der Blätter, des Stamms oder der Wurzeln, abgegeben werden. Der Wirkstoff penetriert nach der Abgabe aus dem hydrophilen Haftschmelzkleber in epidermale und subepidermale Gewebe der Pflanze, tritt in die Stofftransportsysteme der Pflanze über und wird nach der Resorption systemisch in der Pflanze verteilt.

Die systemischen Wirkstoffe können beispielsweise Fungizide, wie Bitertanol, Fenarimol, Triforin, Aluminiumfosetyl oder Tridemorph, Insektizide wie Nicotin, Demeton, Dimethoat, Fenthion oder Menazon, oder Pflanzenhormone wie Auxine, Gibberelline, Cytokinine oder Abscisinsäure sein.

Grundformulierung und Herstellung der erfindungsgemäßen Vorrichtung zur Abgabe von Stoffen aus Haftschmelzklebern mit gleichmäßiger oder ungleichmäßiger Verteilung der Stoffe entsprechend den Merkmalen des Hauptanspruchs werden im folgenden beispielhaft erläutert:

### Beispiel 1:

100 Teile des wasserlöslichen Klebharzes Pantotenylalkohol werden bei 70°C geschmolzen. In die Schmelze werden 30 Teile Polyethylenglycol mit einem Molekulargewicht von 15000 bis zur klaren Schmelze langsam eingerührt.

In die Schmelze werden 10 Teile Natriumalginat und 1,5 Teile abzugebender Stoff, beispielsweise Lidocainhydrochlorid, langsam eingerührt, bis eine gleichmäßige Verteilung erreicht ist.

Der Haftschmelzkleber wird dann mittels Rakelauftrag auf ein geeignetes Substrat, z.B. einen silikonisierten Papierverbund gestrichen.

Nach dem Erkalten wird eine geeignete Rückschicht, z.B. eine dünne Polyesterfolie, auf den Haftschmelzkleber laminiert. Aus dem Laminat werden Vorrichtungen der gewünschten Fläche ausgestanzt.

Der druckempfindliche, hydrophile Haftkleber der so hergestellten erfindungsmäßigen Vorrichtung weist bei Applikation auf die Haut eine ausgezeichnete Anfangsklebrigkeit, während der Tragedauer eine ausgezeichnete Adhäsion und beim Abziehen von der Haut eine ausgezeichnete Kohäsion auf, so daß die Vorrichtung rückstandsfrei von der Haut entfernt werden kann. Zusätzlich zeichnet sich die Vorrichtung dadurch aus, daß der hydrophile Haftschmelzkleber Hautfeuchtigkeit absorbiert, daß er das Entstehen von bakterienwachstumsbegünstigenden Wasserfilmen verhindert, daß er keinerlei Hautreizungen oder allergische Reaktionen verursacht und daß er, bedingt durch die Aufnahme von Hautfeuchtigkeit, an der Grenzfläche zur Haut weich und geschmeidig wird, wodurch er ohne Beschädigung der Hornschicht leicht und schmerzfrei ohne Rückstände von der Haut zu entfernen ist.

In einer anderen bevorzugten Ausführungsform der erfindungsgemäßen Vorrichtung zur Abgabe von Stoffen aus Haftschmelzklebern umfaßt der hydrophile Haftschmelzkleber zur Beeinflußung der thermodynamischen Aktivität des abzugebenden Stoffs 0,1% bis 40% Wasser.

### Beispiel 2 :

20 Teile Pantothenylalkohol werden bei 90°C geschmolzen, in die Schmelze werden 10 Teile Gylcerin, 2 Teile Tween 20, 2 Teile Ethylcellulose und 1,5 Teile Estradiol langsam eingerührt bis eine gleichmäßige Verteilung erreicht ist (Schmelze A).

18 Teile Gelatine, 10 Teile Polyvinylpyrrolidon, 5 Teile Natriumcarboximethylcellulose und 11,5 Teile Saccharose werden bei Raumtemperatur mit 20 Teilen Wasser angeteigt, intensiv homogenisiert bis zur Erreichung einer gleichmäßigen Verteilung und anschließend unter Rühren auf 90°C erhitzt (Schmelze B).

Schmelze B wird bei 90°C langsam in Schmelze A eingerührt bis eine gleichmäßige Verteilung erreicht ist. Der Haftschmelzkleber wird dann mittels Rakelauftrag auf einen silikonisierten Papierverbund gestrichen, und nach dem Erkalten wird eine Polyesterfolie auflaminiert. Aus dem Laminat werden Vorrichtungen der gewünschten Fläche ausgestanzt.

Der druckempfindliche, hydrophile Haftschmelzkleber der so hergestellten erfindungsmäßigen Vorrichtung entspricht bei Applikation auf die Haut den bereits genannten Anforderungen bezüglich Anfangsklebrigkeit, Haftung während der Tragedauer, Kohäsion, Absorption von Hautfeuchtigkeit sowie Hautfreundlichkeit und weist zudem den Vorteil auf, daß im Vergleich zu den bekannten lipophilen transdermalen Systemen durch die Anwesenheit von Wasser die thermodynamische Aktivität von Estradiol in der Klebschicht signifikant beeinflusst wird. Dies erhöht die Möglichkeiten zur Beeinflussung und Steuerung der Estradiol-Freisetzung aus einer derartigen Vorrichtung. Desweiteren weist die beschriebene Formulierung den Vorteil auf, daß, wenn sie zur Abgabe von Estradiol oder anderen Stoffen an Wunden eingesetzt wird, aufgrund des hydrophilen Charakters zum einen eine Interaktion mit Wundexsudat möglich ist und zum anderen durch die Anwesenheit von Wasser in der Formulierung bei fehlender Exsudation der Wunde ein die Heilungsprozesse förderndes, feuchtes Mikroklima in der Wunde geschaffen wird.

In einer weiteren bevorzugten Ausführungsform der erfindungsgemäßen Vorrichtung zur Abgabe von Stoffen aus Haftschmelzklebern umfaßt der hydrophile Haftschmelzkleber zur Beeinflussung der thermodynamischen Aktivität des abzugebenden Stoffs eine lipophile Phase.

### Beispiel 3 :

27 Teile Pantothenylalkohol werden bei 90°C geschmolzen. In der Schmelze werden 12,5 Teile Natriumalginat, 12,5 Teile Gelatine und 1 Teil Chlorophyll langsam eingerührt bis eine gleichmäßige Verteilung erreicht ist (Schmelze A).

Getrennt davon werden 25 Teile Hydroabietylalkohol bei 125°C geschmolzen. In der Hydroabietylalkoholschmelze werden 10 Teile eines Ethylen-Vinylacetat-Copolymers mit einem Vinylacetatgehalt von 28%, 10 Teile eines Polyalkadiens mit Monomerkettenlänge C4 bis C53 und 2 Teile Lidocain langsam bis zur klaren Schmelze bei 120°C eingerührt (Schmelze B). Schmelze B wird untat Rühren auf 90°C gekühlt und bei 90°C langsam in Schmelze A eingerührt, bis eine gleichmäßige Verteilung erreicht ist. Der Haftschmelzkleber wird dann mittels Rakelauftrag auf einen silikonisierten Papierverbund gestrichen; nach dem Erkalten wird eine Polyesterfolie auflaminiert, und aus dem Laminat werden Vorrichtungen der gewünschten Fläche ausgestanzt.

Der druckempfindliche, hydrophile Haftschmelzkleber der so hergestellten erfindungsgemäßen Vorrichtung entspricht bei Applikation auf intakte Haut oder auf Wunden wie die zuvor beschriebene Vorrichtung den genannten Anforderungen an Anfangsklebrigkeit, Haftung während der Tragedauer, Kohäsion, Hautfreundlichkeit und Absorption bzw. Interaktion mit Wundexsudat oder Hautfeuchtigkeit.

Der Vorteil der Kombination der hydrophilen Grundbestandteile der erfindungsgemäßen Vorrichtung mit lipophilen Formulierungsbestandteilen in einem hydrophilen Haftschmelzkleber liegt darin, daß auch lipophile Stoffe, die aus dem Haftschmelzkleber abgegeben werden sollen, in zumindest teilweise gelöster Form vorliegen, was eine wichtige Voraussetzung für die Diffusion des Stoffes im Haftschmelzkleber und damit auch für die Abgabe aus dem Haftschmelzkleber ist.

Die lipophile Phase der erfindungsgemäßen Vorrichtung kann, wie im zuvor beschriebenen Beispiel, unzerteilt und kohärent sein, sie kann aber auch zerteilt und inkohärent in Form von kleinen Partikeln oder Tröpfchen vorliegen.

Als Bestandteile der lipophilen Phase können Homo-, Co- oder Blockpolymere wie z.B. Polyamide, Polyester, Polycaprolactame, Polycaprolacton, Ethylen-Vinylacetat-Copolymere (EVA), Ethylen-Ethylacrylat-Copolymere (EEA), Polyvinylether, Poly(meth)acrylate, Polyvinylacetale, Polyvinylacetate, Styrol-Butadien-Blockpolymere, Isopren-Blockpolymere, Polyurethane, Ethylcellulose, Celluloseacetat-Butyrat, Synthesekautschuke (z.B. Neopren-Kautschuk), Polyisobutylen, Butylgummi und Acrylnitril-Butadien-Mischpolymerisate oder Harze, wie z.B. Epoxidharze, Melaminharze, Phenol-Formaldehyd-Harze und Resorcin-Formaldehyd-Harze verwendet werden, wobei auch unter anderem die nachfolgend genannten modifizierten Harze eingesetzt werden können: Hydriertes Kolophonium, polymerisiertes Kolophonium, dimerisierte Harzsäuren, disproportioniertes Kolophonium, Methylester von Kolophonium, Glycerinester von hydriertem Kolophonium, Methylester von hydriertem Kolophonium, Pentalester, Triethylenglycolester von hydriertem Kolophonium, Hydroabietylalkohol und dessen Derivate, Glycerinester, Di-Triolester und Pentalester von Harzsäuren, Pentalester von polymerisiertem Kolophonium, Pentalester von dimerisiertem Kolophonium, Glycerinester von dimerisiertem Kolophonium, Ester von Maleinsäure- oder Phenol-modifiziertem Kolophonium, aromatische und aliphatische Kohlenwasserstoffharze, hydrierte Harze, Polyterpenharze und modifizierte Terpenharze.

Die lipophile Phase kann aber auch aus natürlichen, halbsynthetischen oder synthetischen Fetten und Ölen wie Olivenöl, Ricinusöl, Erdnusöl, Sojaöl, Leinöl, Sesamöl, Jojobaöl, Avocadoöl, hydriertes Erdnußöl, hydriertes Ricinusöl, Triglyceridgemische (Miglylol®, Softisan®) oder Silikonöle, natürlichen, halbsynthetischen oder synthetischen Wachsen wie Bienenwachs, Wollwachs, Erdwachs, Walrat, Ölsäureoleylester, Isopropylpalmitat, Isopropylmyristat, Ethyloleat, Cetylpalmitat oder Cetylstearat, Fettalkohole wie Dodecylalkohol oder Cetylalkohol, Fettsäuren wie Myristinsäure, Ölsäure oder Linolsäure, propoxylierte, ethoxylierte oder sulfatierte Fettalkohole, Fettsäurealkylamide, Fettsäure-Eiweiß-Kondensationsprodukte, Phospholipide, Sterine oder Kohlenwasserstoffe wie Paraffine oder Paraffinöle bestehen. Liegt die lipophile Phase in zerteilter Form vor, so werden zur Stabilisierung der zerteilten Phase der erfindungsgemäßen Zubereitung Hilfsstoffe wie beispielsweise Emulgatoren und/oder Schutzkolloide zugesetzt.

Zusätzlich zu den bereits genannten Hilfsstoffen kann die Vorrichtung zur Abgabe von Stoffen aus hydrophilen Haftschmelzklebern als Hilfsstoffe enthalten:
- Feuchthaltemittel wie bspw. Glycerin, Sorbitol, Polyethylenglykol, Polypropylenglykol
- Weichmacher wie bspw. Citronensäureester, Weinsäurester oder Glycerinester
- Penetrationsbeschleuniger wie bspw. Alkylsulfate, Alkylsulfonate, Alkaliseifen, fettsaure Salze von mehr wertigen Metallen, Betaine, Aminoxide, Fettsäureester, Mono-, Di- oder Triglyceride, langkettige Alkohole, Sulfoxide, Nicotinsäureester, Salicylsäure, N-Methyl pyrrolidon, 2-Pyrrolidon oder Harnstoff.
- Konservierungsmittel wie bspw. p-Cl-m-Kresol, Phenylethylakohol, Phenoxiethylalkohol, Chlorbutanol, 4-Hydroxibenzoesäuremethylester, 4-Hydroxibenzoesäurepropylester, Benzalkoniumchlorid, Cetylpyridiniumchlorid, Chlorhexidindiacetat oder -gluconat, Ethanol oder Propylenglykol.
- Desinfektionsmittel wie bspw. Halogene wie Polyvidon-Jod, Halogenverbindungen wie Natriumhypochlorid oder Tosylchlorid-Natrium, Oxidationsmittel wie Wasserstoffperoxid oder Kaliumpermanganat, Arylquecksilberverbindungen wie Phenylmercuriborat oder Merbromin, Alkylquecksilberverbindungen wie Thiomersal, Organozinnverbindungen wie Tri-n-butyl-Zinnbenzoat, Silberweißverbindungen wie Silberweißacetyltannat, Alkohole wie Ethanol, n-Propanol oder Isopropanol, Phenole wie Thymol, o-Phenylphenol, 2-Benzyl-4-Chlorphenol, Hexachlorophen oder Hexylresorcin oder organische Stickstoffverbindungen wie 8-Hydroxichinolin, Chlorquinaldol, Clioquinol, Ethacridin, Hexetidin, Chlorhexidin oder Ambazon,
- pH-Regulatoren wie bspw. Glycerinpuffer, Citratpuffer, Boratpuffer, Phosphatpuffer oder Citronensäure-Phosphat-Puffer
- Antioxidantien wie bspw. Ascorbinsäure, Ascorbylpalmitat, Tocopherolacetat, Propylgallat, Butylhydroxianisol oder Butylhydroxitoluol,
- Wirkstoffstabilisatoren wie Mannitol, Glucose, Lactose, Fructose, Saccharose
- Duftstoffe, Farbstoffe, Reinigungsstoffe, Körperpflegestoffe
- Emulsionstabilisatoren wie z.B. nichtionogene Emulgatoren, amphotere Emulgatoren, kationaktive Emulgatoren und anionaktive Emulgatoren
- Füllstoffe wie z.B. mikrokristalline Cellulose, Aluminiumoxid, Zinkoxid, Titanoxid, Talkum, Siliciumdioxid, Magnesiumsilikat, Magnesium-Aluminiumsilikat, Kaolin, hydrophobe Stärke, Calciumstearat oder Calciumphosphat.

Neben dem in den vorgenannten Beispielen erwähnten Rakelauftragsverfahren kann die Verarbeitung der wirkstoffabgebenden hydrophilen Haftschmelzklebeschicht auch nach anderen, dem Fachmann bekannten Verfahren wie beispielsweise Walzenauftrag, Extrusion, Gießen, Aufsprühen oder Druckverfahren erfolgen.

Ein Grenzwert für die Verarbeitbarkeit der hydrophilen Haftschmelzkleber bei vielen dieser Verfahren ist bei einer Viskosität im Bereich von etwa 80 000 Pa gegeben.

Falls die mit dem Kleber zu behandelnde Unterlage - eine Komponente der Vorrichtung - durch die Temperatur des heiß aufgetragenen Klebers beschädigt werden könnte, sei es durch Zersetzung, Reaktion oder partielles Schmelzen, kann eine gekühlte Unterlage eingesetzt werden. Die Kühlung kann durch an sich bekannte Verfahren erfolgen, wie durch Einbringung kalter inerter Gase oder das Kontaktieren mit einer Kühlfläche.

Beim Einsatz leicht flüchtiger und/oder thermisch instabiler abzugebender Stoffe können die nachfolgend genannten besonderen Maßnahmen zur Verarbeitung angezeigt sein:
A: das Arbeiten bei möglichst tiefen Temperaturen
B: die Erhöhung des Außendrucks, um die Verdampfung herabzusetzen
C: die Sättigung des Dampfraums über der Schmelze mit dem dampfförmigen Stoff
D: das Arbeiten mit entsprechend den Vorgaben kleinstmöglicher Menge an flüchtigem Stoff in der Schmelze.

Selbstverständlich sind diese Maßnahmen, wie bspw. das Arbeiten in einer gekapselten Anlage durch die dem Fachmann durch den Einsatzzweck der herzustellenden Vorrichtung als auch die stofflichen Gegebenheiten bekannten Gesetzmäßigkeiten limitiert.

Erfindungsgemäße Vorrichtungen, bei denen der wirkstoffabgebende Teil der Vorrichtung hydrophile Haftschmelzkleber enthält, können nach allen dem Fachmann bekannten Pflasterkonstruktionen aufgebaut sein. Grundtypen derartiger Konstruktionen sind beispielsweise das Membransystem und das Matrixsystem.

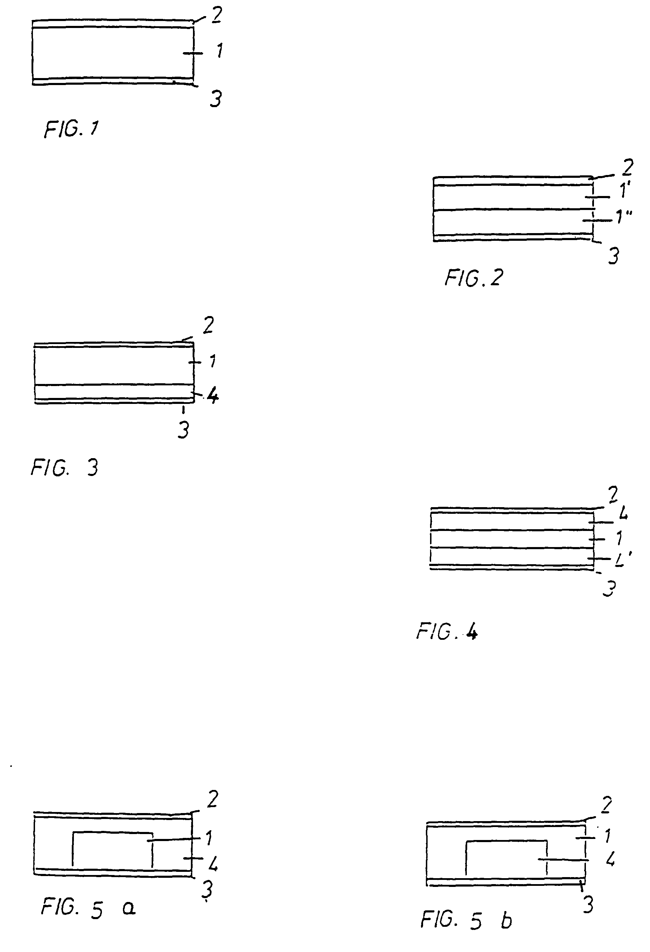

Ein Membransystem enthält mindestens 5 Teile: eine flexible Rückschicht, einen wirkstoffhaltigen Haftschmelzkleber, eine Membran zu Steuerung der Wirkstofffreisetzung, eine auf die Membran laminierte Klebschicht zur Befestigung des Systems auf der Haut sowie einer ablösbaren Schutzschicht, die die zur Haut gewandte, klebende Fläche des Pflasters abdeckt. Ein Matrixsystem (Fig. 1) enthält in einfachster weise drei Teile: eine flexible Rückschicht, einen wirkstoffhaltigen, hydrophilen Haftschmelzkleber und eine ablösbare Schutzschicht, die die zur Haut gewandte, klebende Fläche der Vorrichtung abdeckt.

Ein Verfahren zur Herstellung einer erfindungsgemäßen Vorrichtung vom Typ Matrixsystem beinhaltet kontinuierliches oder diskontinuierliches Aufbringen abzugebenden Stoff enthaltenden geschmolzenen hydrophilen Haftschmelzklebers bei einer Temperatur des Haftschmelzklebers zwischen 40 und 150°C, bevorzugt 80 bis 120°C auf eine flexible Rückschicht und ggf. Anbringen des Schutzschichtmaterials.

Ein weiteres Verfahren beinhaltet kontinuierliches oder diskontinuierliches Aufbringen abzugebenden Stoff enthaltenden geschmolzenen hydrophilen Haftschmelzklebers bei einer Temperatur des Haftschmelzklebers zwischen 40 und 150°C, bevorzugt 80 bis 120°C auf ein Schutzschichtmaterial und ggf. Anbringen der flexiblen Rückschicht.

Der hydrophile Haftschmelzkleber kann bspw. in Schichtform oder in einzelnen Bereichen entsprechend einem vorherbestimmten Muster auf die Schutzschicht oder die Rückschicht aufgetragen werden.

Geeignete Materialien für die flexible Rückschicht sind beispielsweise Polyester, Polyamid, Polyethylen, Polypropylen, Polyurethan, Polyvinylchlorid, und zwar sowohl als sogenannte Solofolien als auch als Sandwichfolien in Kombination von Folien aus verschiedenen dieser Kunststoffe. Diese Folien können außerdem mit Aluminium bedampft bzw. mit einer Aluminiumfolie kaschiert sein.

Geeignete Materialien für die ablösbare Schutzschicht sind beispielsweise Polyester, Polyethylen, Polypropylen sowie Papiere, die mit diesen Materialien beschichtet und ggf. mit Aluminium bedampft bzw. mit einer Aluminiumfolie kaschiert wurden. Außerdem sind die Folien bzw. Papiere mit Silikon beschichtet, um ihnen die wiederablösbaren Eigenschaften zu verleihen.

Je nach Anwendungszweck, falls bspw. der abzugebende Stoff durch die Rückschicht abgegeben werden soll, wie es bspw. bei ätherischen Ölen sein kann, kann der Haftschmelzkleber mit einer für den/die abzugebenden Stoff(e) durchlässigen Rückschicht ausgerüstet sein, während bei der Ausführungsform der Vorrichtung als transdermales System, bei dem der Stoff nur an die Haut abzugeben ist, eine für den abzugebenden Stoff undurchlässige Rückschicht bevorzugt ist.

Während des Schmelzens der Formulierungsbestandteile des hydrophilen Haftschmelzklebers kann in die Masse Luft mit eingerührt werden, so daß der nach dem Ausstreichen oder Ausgießen erhaltene hydrophile Haftschmelzkleber eine poröse Struktur hat. Herstellung und Stabilisierung der porösen Struktur können z.B. durch die Zugabe von Schäumungsmitteln wie Saponinen, Alginsäureestern, Aminoxiden oder Fettaminoxiden unterstützt werden.

Die Zusammensetzung der erfindungsgemäßen Vorrichtng insgesamt oder auch nur des hydrophilen Haftschmelzklebers kann so gewählt werden, daß das hydrophile Haftschmelzkleber biologisch abbaubar ist, wobei unter biologischem Abbau beispielsweise die Einwirkung von UV-Licht, die Einwirkung von Mikroben oder die Einwirkung von Enzymen verstanden wird.

Die erfindungsgemäße Vorrichtung wie auch der hydrophile Haftschmelzkleber können mehrschichtig aufgebaut sein. Es können beispielsweise zumindest zwei hydrophile Haftschmelzkleber nach bekanntem Verfahren zu einem Verbund zusammengefügt werden (Fig. 2). Ebenso ist es möglich, einen oder mehrere hydrophile Haftschmelzkleber mit einem oder mehreren lipophilen Elementen zu einem Verbund beliebigen Aufbaus zusammenzufügen (Fig. 3/4). Solche Elemente können beispielsweise als Steuerelemente, Speicherelemente, durchlässige oder undurchlässige Trennelemente dienen. Die einzelnen Elemente des Verbunds können dabei in sich zusammenhängend und kohärent sein, sie können aber auch in Segmente zerteilt sein, die von dem darüber oder darunter liegenden Element umgeben oder umschlossen werden (Fig. 5/6). So kann beispielsweise eine lipophile Kleberschicht zusammenhängend oder punkt- bzw. rautenförmig zerteilt auf einem hydrophilen Haftschichtkleber aufgebracht sein, wobei der hydrophile Haftschichtkleber die Zwischenräume zwischen den lipophilen Segmenten ausfüllen kann (Fig. 7). In einer anderen Ausführungsform wird der hydrophile Haftschichtkleber mit textilen Geweben, Fasergeflechten und/oder natürlichen oder synthetischen Schäumen kombiniert. Bei diesem Mehrschichtverbund können die einzelnen Schichten nach bekannten Verfahren getrennt hergestellt und anschließend zusammengebracht werden. Die Schichten können sich aber auch gegenseitig durchdringen, so beispielsweise, wenn die geschmolzene Masse des hydrophilen Haftschmelzklebers vor der Filmbildung auf ein Gewebe, ein Fasergeflecht oder einen Schaum gegeben wird und die Filmbildung erst nach Einziehen der Masse in das Gewebe oder den Schaum erfolgt.

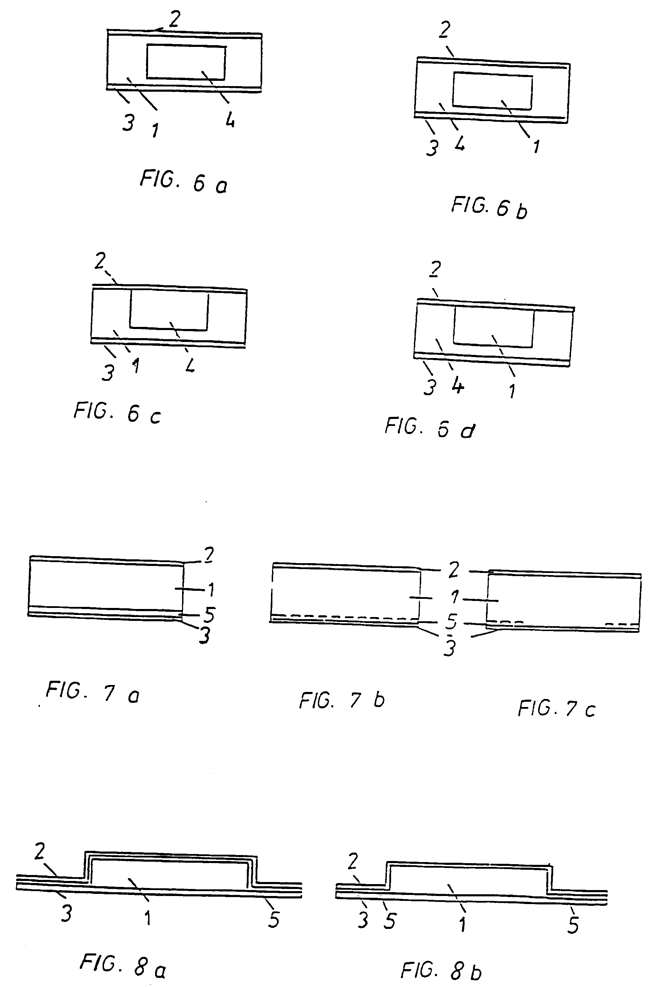

Die Befestigung der erfindungsgemäßen Vorrichtung am Anwendungsort erfolgt in der Regel durch den hydrophilen Haftschmelzkleber der Vorrichtung. Eine andere Möglichkeit zur Befestigung der erfindungsgemäßen Vorrichtung am Anwendungsort besteht darin, daß die Rückschicht größere Flächenabmessungen als der Teil, der aus hydrophilem Haftschmelzkleber besteht, hat und daß zumindest die überstehenden Teile der Rückschicht mit einer zusätzlichen Klebeschicht ausgerüstet sind (Fig. 8).

Die letztgenannten Ausführungsformen der erfindungsgemäßen Vorrichtung, bei denen der hydrophile Haftschmelzkleber mehrschichtig, mehrteilig oder in Kombination mit Trenn- und Steuerelementen vorliegt, bieten den Vorteil, daß Teile unterschiedlicher Zusammensetzung miteinander kombiniert werden können. Dabei soll durch Verwendung unterschiedlicher Hilfsstoffe oder unterschiedlicher Wirkstoffkonzentrationen in den einzelnen Elementen ein Freisetzungsverhalten des Wirkstoffs oder der Wirkstoffe erreicht werden, das für die Anwendung notwendig und durch einschichtigen Aufbau des hydrophilen Haftschmelzklebers z.B. nicht zu erzielen ist.

Nachfolgend werden die der Verdeutlichung der Erfindung dienenden Fig. 1 bis 8 erläutert:
Fig. 1 stellt einen hydrophilen Haftschmelzkleber (1) dar, der auf einer Seite mit einer undurchlässigen Rückschicht (2) und auf der gegenüberliegenden Seite mit einer ablösbaren, undurchlässigen Schutzschicht (3) versehen ist.
Fig. 2 beschreibt einen mehrschichtigen hydrophilen Haftschmelzkleber (Laminat), in dem der hydrophile Haftschmelzkleber (1) in zwei Schichten (1',1'') unterteilt ist.
Fig. 3 zeigt den wirkstoffhaltigen hydrophilen Haftschmelzkleber (1), der auf der dem Substrat zugewandten Seite mit einem wirkstofffreien, lipophilen Element (4), das als Steuermembran dient, kombiniert ist.
Fig. 4 stellt ebenfalls einen Mehrschichtverbund dar, in dem ein wirkstoffhaltiger, hydrophiler Haftschmelzkleber (1) sandwichartig von zwei lipophilen Elementen (4',4'') umgeben ist. Abgedeckt wird diese Anordnung wieder durch Rückschicht (2) und Schutzschicht (3).
Fig. 5a und 5b zeigen eine Ausführungsform des hydrophilen Haftschmelzklebers (1), in dem dieser zumindest teilweise von lipophilem Element (4) umgeben ist, oder dieser selbst zumindest teilweise ein lipophiles Element umgibt. Beide Elemente können wirkstoffhaltig sein.
Fig. 6a zeigt eine weitere Ausführungsform, in der der hydrophile Haftschmelzkleber (1), der als wirkstoffhaltiges Steuerelement wirkt, einen zusätzlichen lipophilen Wirkstoffspeicher (4) umschließt; in Fig. 6b ist der als Speicherelement wirkende hydrophile Haftschmelzkleber (1) in einem lipophilen, als Steuerelement wirkenden Film (4) vollständig eingebettet, lipophiles Speicherelement (4) bzw. hydrophiler Haftschmelzkleber (1) als Speicher können auch mit einer Oberfläche direkt an die Rückschicht (2) angrenzen (Fig. 6c und 6d).
Fig. 7a, 7b und 7c zeigen Ausführungsformen eines hydrophilen Haftschmelzklebers (1) mit Klebstoffschicht (5), wobei diese Klebstoffschicht kontinuierlich (Fig. 7a) oder diskontinuierlich, d.h. unterbrochen den gesamten hydrophilen Haftschmelzkleber (Fig. 7b) oder Teile des hydrophilen Haftschmelzklebers (Fig. 7c) bedeckt.
Fig. 8a und 8b zeigen Vorrichtungen, bei denen ein hydrophiler Haftschmelzkleber (1) mit einer Schutzschicht (3) und mit einer Rückschicht (2), die größere Flächenabmessungen als der hydrophile Haftschmelzkleber (1) aufweist und die ganz (Fig. 8a) oder teilweise (Fig. 8b) mit einem Klebstofffilm (5) beschichtet ist, bedeckt ist.

Zusätzlich zu den bereits dargestellten Beispielen 1 bis 3, die der grundsätzlichen Erläuterung der erfindungsgemäßen Vorrichtung zur Abgabe von Stoffen aus hydrophilen Haftschmelzklebern dienen, sollen die folgenden Beispiele Formulierungsmöglichkeiten, Ausführungsformen und Anwendungsmöglichkeiten der im Hauptanspruch genannten Vorrichtung beschreiben, wobei diese Auflistung keinesfalls umfassend sein kann:

### Beispiel 4 :

25 Teile Pantothenylalkohol werden bei 90°C geschmolzen. In die Schmelze werden 10 Teile Natriumalginat und 10 Teile Gelatine langsam eingerührt bis eine gleichmäßige Verteilung erreicht ist (Schmelze A).

Getrennt davon werden 25 Teile Hydroabietylalkohol bei 125°C geschmolzen. In der Hydroabietylalkoholschmelze werden 10 Teile eines Ethyl-Vinylacetat-Copolymers mit einem Vinylacetatgehalt von 28%, 10 Teile eines Polyalkadiens mit Monomerkettenlänge C4 bis C5 und 10 Teile Acetylsalicylsäure langsam bis zur klaren Schmelze bei 120°C eingerührt (Schmelze B).

Schmelze B wird unter Rühren auf 90°C gekühlt und bei 90°C langsam in Schmelze A eingerührt, bis eine gleichmäßige Verteilung erreicht ist. Der Haftschmelzkleber wird dann mittels Rakelauftrag mit einem Flächengewicht von 300 g/m² auf einen silikonisierten Papierverbund gestrichen; nach dem Erkalten wird eine Polyesterfolie auflaminiert, und aus dem Laminat werden Vorrichtungen mit 30 cm² Fläche ausgestanzt; die Vorrichtungen enthalten 90 mg Acetylsalicylsäure.

### Beispiel 5 :

20 Teile Pantothenylalkohol werden bei 90°C geschmolzen. In die Schmelze werden 10 Teile Glycerin, 4 Teile Ethylcellulose, 4 Teile Lidocain und 4 Teile Prilocain langsam eingerüht bis eine gleichmäßige Verteilung erreicht ist (Schmelze A).

18 Teile Gelatine, 10 Teile Polyvinylpyrrolidon, 5 Teile Natriumcarboximethylstärke und 5 Teile Saccharose werden bei Raumtemperatur mit 20 Teilen Wasser angeteigt, intensiv homogenisiert bis zur Erreichung einer gleichmäßigen Verteilung und anschließend unter Rühren auf 90°C erhitzt (Schmelze B).

Schmelze B wird bei 90°C langsam in Schmelze A eingerührt, bis eine gleichmäßige Verteilung erreicht ist. Der Haftschmelzkleber wird dann mittels Rakelauftrag mit einem Flächengewicht von 500 g/m² auf einen silikonisierten Papierverbund gestrichen; nach dem Erkalten wird eine Polyesterfolie auflaminiert. Aus dem Laminat werden Vorrichtungen mit 25 cm² Fläche ausgestanzt; die Vorrichtungen enthalten 50 mg Lidocain und 50 mg Prilocain.

### Beispiel 6 :

25 Teile Pantothenylalkohol werden bei 90°C geschmolzen. In die Schmelze werden 12,5 Teile Natriumalginat und 12,5 Teile Gelatine langsam eingerührt bis eine gleichmmäßige Verteilung erreicht ist (Schmelze A).

Getrennt davon werden 25 Teile Hydroabietylalkohol bei 125°C geschmolzen. In der Hydroabietylalkoholschmelze werden 10 Teile eines Ethylen-Vinylacetat-Copolymers mit einem Vinylacetatgehalt von 28%, 10 Teile eines Polyalkadiens mit Monomerkettenlänge C4 bis C5, 2,5 Teile Lidocain und 2,5 Teile Prilocain langsam bis zur klaren Schmelze bei 120°C eingerührt (Schmelze B).

Schmelze B wird unter Rühren auf 90°C langsam in Schmelze A eingerührt, bis eine gleichmäßige Verteilung erreicht ist. Der Haftschmelzkleber wird dann mittels Rakelauftrag mit einem Flächengewicht von 500 mg/m² auf einen silikonisierten Papierverbund gestrichen; nach dem Erkalten wird eine Polyesterfolie auflaminiert, und aus dem Laminat werden Vorrichtungen mit 25 cm² ausgestanzt; die Vorrichtungen enthalten 31,25 mg Lidocain und 31,25 mg Prilocain.

### Beispiel 7 :

20 Teile Pantothenylalkohol werden bei 90°C geschmolzen. In die Schmelze werden 10 Teile Glycerin, 1 Teil Ethylcellulose und 5 Teile Tabakpulver langsam eingerührt, bis eine gleichmäßige Verteilung erreicht ist (Schmelze A).

18 Teile Gelatine, 10 Teile Polyvinylpyrrolidon, 5 Teile Natriumcarboximethylstärke und 11 Teile Saccharose werden bei Raumtemperatur mit 20 Teilen Wasser angeteigt, intensiv homogenisiert bis zur gleichmäßigen Verteilung und anschließend unter Rühren auf 90°C erhitzt (Schmelze B).

Schmelze B wird bei 90°C langsam in die Schmelze A eingerührt bis eine gleichmäßige Verteilung erreicht ist. Der Haftschmelzkleber wird dann mittels Rakelauftrag mit einem Flächengewicht von 600 g/m² auf einen silikonisierten Papierverbund gestrichen; nach dem Erkalten wird eine Polyesterfolie auflaminiert. Aus dem Laminat werden Vorrichtungen mit 20 cm² Fläche ausgestanzt; die Vorrichtungen enthalten 60 mg Tabakpulver.

### Beispiel 8 :

50 Teile Pantothenylalkohol werden bei 90°C geschmolzen. In der Schmelze werden 25 Teile Polyethylenglycol von einem Molekulargewicht von 15 000, 20 Teile Natriumalginat und 5 Teile wäßrige Chlorhexidindiglukonatlösung langsam eingerührt bis eine gleichmäßige Verteilung erreicht ist.

Der Haftschmelzkleber wird dann mittels Rakelauftrag mit einem Flächengewicht von 300 g/m² auf einen silikonisierten Papierverbund gestrichen; nach dem Erkalten wird eine Polyesterfolie auflaminiert. Aus dem Laminat werden Vorrichtungen mit 200 cm² Fläche ausgestanzt, die 300 mg Chlorhexidindiglukonatlösung enthalten. Die Vorrichtung wird lokal eingesetzt zur Hautdesinfektion bei bakteriell bedingten Hauterkrankungen oder zur Desinfektion infizierter Wunden.

### Beispiel 9 :

20 Teile Pantothenylalkohol werden bei 90°C geschmolzen. In die Schmelze werden 16 Teile Natriumalginat, 9 Teile Salicylsäure und 2 Teile Ethylcellulose eingerührt, bis eine gleichmäßige Verteilung erreicht ist (Schmelze A).

Getrennt davon werden 28 Teile Hydroabietylalkohol, 15 Teile eines Ethylen-Vinylacetat-Copolymers mit einen Vinylacetatgehalt von 28% und 10 Teile eines Polyalkadiens mit Monomerkettenlänge C4 bis C5 bei 125°C geschmolzen (Schmelz. B).

Schmelze B wird unter Rühren auf 90°C gekühlt und bei 90°C langsam in Schmelz. A eingerührt, bis eine gleichmäßige Verteilung erreicht ist. Der Haftschmelzkleber wird dann mittels Rakelauftrag mit einem Flächengewicht von 1400 g/m² auf einen silikonisierten Papierverbund gestrichen; vor dem Erkalten wird auf die noch weiche Masse ein offenzelliger Polyurethan-Schaum von 2 mm, auf den rückseitig eine 20 µm starke Polyurethanfolie gesiegelt ist, unter leichtem Druck aufgelegt.

Nach dem Erkalten werden aus dem Verbund ovale Vorrichtungen von 15 cm² Fläche ausgestanzt. Diese Vorrichtungen, die einen Haftschmelzkleber-Schaumstoff-Verbund umfassen, werden wegen der keratolytischen Wirkung der im Haftschmelzkleber enthaltenen Salicylsäure beispielsweise zur lokalen Behandlung von Hübneraugen verwendet. Durch den Verbund mit Schaumstoff wird dabei gleichzeitig der Druck auf die zu behandelnde Stelle reduziert.

### Beispiel 10 :

20 Teile Pantothenylalkohol werden bei 90°C geschmolzen. In die Schmelze werden 10 Teile Glycerin und 1 Teil Ethylcellulose langsam eingerührt bis eine gleichmäßige Verteilung erreicht ist (Schmelze A).

18 Teile Gelatine, 10 Teile Polyvinylpyrrolidon, 5 Teile Natriumcarboximethylstärke, 10 Teile Saccharose, 2 Teile Johanniskrauttinktur, 2 Teile Sonnenhutkrauttinktur und 2 Teile Kamillenextrakt werden bei Raumtemperatur mit 20 Teilen Wasser angeteigt, intensiv homogenisiert bis zur Erreichung einer gleichmäßigen Verteilung und anschließend unter Rühren auf 90°C erhitzt (Schmelze B).

Schmelze B wird bei 90°C langsam in Schmelze A eingerührt bis eine gleichmäßige Verteilung erreicht ist. Der Haftschmelzkleber wird dann mittels Rakelauftrag mit einem Flächengewicht von 600 g/m² auf eine silikonisierte HDPE-Folie gestrichen; nach dem Erkalten wird eine Polyesterfolie auflaminiert. Aus dem Haftschmelzkleber-Polyester-Verbund werden kreisrunde Stücke mit einer Fläche von 10 cm² ausgestanzt. Diese Stücke werden mit der Polyesterseite auf die Klebschicht einer acrylatbeschichteten Polyesterfolie laminiert. Nach dem Abziehen der silikonisierten HDPE-Folie wird ein silikonisierter Papierverbund zukaschiert, und es werden kreisrunde Vorrichtungen mit einer Fläche von 25 cm² so ausgestanzt, daß sich der hydrophile Haftschmelzkleber zentral in der Mitte der Vorrichtung befindet. Die Vorrichtung besteht somit aus einer Polyester Rückschicht, einer Klebschicht zur Befestigung auf der Haut, einer Polyester Trennschicht zur Verhinderung des Einwanderns von Stoffen aus dem hydrophilen Haftschmelzkleber in die lipophile Klebschicht, dem tinktur- bzw. extrakthaltigen hydrophilen Haftschmelzkleber und einer vor der Anwendung zu entfernenden Schutzschicht.

Die Vorrichtung wird beispielsweise lokal zur Behandlung von Verbrennungen ersten Grades, stumpfen Verletzungen und schlecht heilenden, oberflächlichen Wunden eingesetzt.

### Beispiel 11 :

18 Teile Pantothenylalkohol werden bei 90°C geschmolzen. In die Schmelze werden 8 Teile Glycerin und 4 Teile Ethylcellulose langsam eingerührt bis eine gleichmäßige Verteilung erreicht ist (Schmelze A).

20 Teile Gelatine, 10 Teile Polyvinylpyrrolidon, 5 Teile Natriumcarboxymethylstärke, 10 Teile Saccharose und 5 Teile einer 4%igen Kollagendispersion werden bei Raumtemperatur mit 20 Teilen Wasser angeteigt, intensiv homogenisiert bis zur Erreichung einer gleichmäßigen Verteilung und anschließend unter Rühren auf 90°C erhitzt (Schmelze B).

Schmelze B wird bei 90°C langsam in die Schmelze A eingerührt bis eine gleichmäßige Verteilung erreicht ist. Der Haftschmelzkleber wird dann mittels Rakelauftrag mit einem Flächengewicht von 1500 g/m² auf einen silikonisierten Papierverbund gestrichen; nach dem Erkalten wird eine Polyurethanfolie auflaminiert. Aus dem Laminat werden Vorrichtungen mit 100 cm² ausgestanzt; die 30 mg reines Kollagen enthalten. Die Vorrichtung wird eingesetzt zur Abgabe des heilungsfördernden Kollagens aus dem hydrophilen Haftschmelzkleber an schlecht heilende Problemwunden, wie beispielsweise Beingeschwüre.

### Beispiel 12 :

20 Teile Pantothenylalkohol werden bei 90°C geschmolzen. In die Schmelze werden 18 Teile Natriumcarboximethylcellulose langsam eingerührt bis eine gleichmäßige Verteilung erreicht ist (Schmelze A).

Getrennt davon werden 32 Teile Hydroabietylalkohol bei 125°C geschmolzen. In der Hydroabietylschmelze werden 16 Teile eines Ethylen-Vinylacetat-Copolymers mit einem Vinylacetatgehalt von 28%, 10 Teile eines Polyalkadiens mit Monomerkettenlänge C4 bis C5, 2 Teile Ethylcellulose, 1 Teil Kamillenöl und 1 Teile Chlorophyll langsam bis zur klaren Schmelze bei 120°C eingerührt (Schmelze B).

Schmelze B wird unter Rühren auf 90°C gekühlt und bei 90°C langsam in Schmelze A eingerührt bis eine gleichmäßige Verteilung erreicht ist. Der Haftschmelzkleber wird dann mittels Rakelauftrag mit einem Flächengewicht von 1000 g/m² auf einen silikonisierten Papierverbund gestrichen; nach dem Erkalten wird eine Polyurethanfolie auflaminiert, und aus dem Laminat werden Vorrichtungen mit 25 cm² Fläche ausgestanzt; die Vorrichtungen, die 25 mg Kamillenöl und 25 mg Chlorophyll enthalten, werden beispielsweise zur Behandlung von Drucketellen und wundgescheuerten Stellen eingesetzt.

### Beispiel 13 :

20 Teile Pantothenylalkohol werden bei 90°C geschmolzen. In die Schmelze werden 21 Teile Natriumcarboximethylstärke und 2 Teile eines glykolischen Extrakts von Fucus vesicolosus eingerührt bis eine gleichmäßige Verteilung erreicht ist (Schmelze A).

Getrennt davon werden 30 Teile Hydroabiethylalkohol, 15 Teile eines Ethyl-Vinylacetat-Copolymers mit einem Vinylacetat-Gehalt von 28%, 10 Teile eines Polyalkadiens mit Monomerkettenlänge C4 bis C5, 1 Teil Ethylcellulose und 1 Teil Vitamin E bei 125°C geschmolzen (Schmelze B).

Schmelze B wird unter Rühren auf 90° gekühlt und bei 90°C langsam in Schmelze A eingerührt bis eine gleichmäßige Verteilung erreicht ist. Der Haftschmelzkleber wird dann mittels Rakelauftrag mit einem Flächengewicht von 300 g/m² auf eine silikonisierte Polyesterfolie gestrichen; nach dem Erkalten wird eine Polyurethanfolie auflaminiert, und aus dem Laminat werden Vorrichtungen mit 10 cm² Fläche ausgestanzt; die Vorrichtungen, die 6 mg Fucus-Extrakt und 3 mg Vitamin E enthalten, werden beispielsweise lokal verwendet zur Behandlung von Hautpartien, die durch Sonneneinwirkung oder Umweltgifte geschädigt sind, um Feuchtigkeit in der Haut zu binden und einen effektiven Zellschutz gegen äußere Einflüsse zu erzielen.

### Beispiel 14 :

20 Teile Pantothenylalkohol werden bei 90°C geschmolzen. In die Schmelze werden 10 Teile Glycerin und 1 Teil Ethylcellulose langsam eingerührt, bis eine gleichmäßige Verteilung erreicht ist (Schmelze A).

18 Teile Gelatine, 10 Teile Polyvinylpyrrolidon, 5 Teile Natriumcarboxymethylstärke, 10 Teile Saccharose, 3 Teile Arnikatinktur und 3 Teile Ringelblumentinktur werden bei Raumtemperatur mit 20 Teilen Wasser angeteigt, intensiv homogenisiert bis zur Erreichung einer gleichmäßigen Verteilung und anschließend unter Rühren auf 90°C erhitzt (Schmelze B).

Schmelze B wird bei 90° langsam in Schmelze A eingerührt bis eine gleichmäßige Verteilung erreicht ist. Der Haftschmelzkleber wird dann mittels Rakelauftrag mit einem Flächengewicht von 400 g/m² auf einen silikonisierten Papierverbund gestrichen; nach Erkalten wird eine Polyesterfolie auflaminiert. Aus dem Laminat werden Vorrichtungen mit 50 cm² Fläche ausgestanzt, die 60 mg Arnikatinktur und 60 mg Ringelblumentinktur enthalten. Die Vorrichtungen werden beispielsweise verwendet zur intradermalen Behandlung von Verstauchungen, Prellungen und Blutergüssen.

### Beispiel 15 :

19 Teile Pantothenylalkohol werden bei 90°C geschmolzen. In die Schmelze werden 8 Teile Glycerin und 4 Teile Ethylcellulose langsam eingerührt bis eine gleichmäßige Verteilung erreicht ist (Schmelze A).

20 Teile Gelatine, 10 Teile Polyvinylpyrrolidon, 5 Teile Natriumcarboximethylstärke, 9 Teile Saccharose und 5 Teile wässriger Extrakt aus grünem Tee werden bei Raumtemperatur mit 20 Teilen Wasser angeteigt, intensiv homogenisiert bis zur Erreichung einer gleichmäßigen Verteilung und anschließend unter Rühren auf 90°C erhitzt (Schmelze B).

Schmelze B wird bei 90°C langsam in Schmelze A eingerührt bis eine gleichmäßige Verteilung erreicht ist. Der Haftschmelzkleber wird dann mittels Rakelauftrag mit einem Flächengewicht von 400 g/m² auf eine silikonisierte Polyesterfolie gestrichen nach dem Erkalten wird eine Polyurethanfolie auflaminiert. Aus dem Laminat werden Vorrichtungen mit 100 cm² Fläche ausgestanzt. Die Vorrichtungen, die 20 mg Tee-Extrakt enthalten, werden bei Applikation auf die Haut verwendet zur Erzielung einer kreislaufstimulierenden Wirkung durch transdermalen Abgabe von Coffein, dem Hauptinhaltsstoffs des Extrakts.

### Beispiel 16 :

20 Teile Pantothenylalkohol werden bei 90°C geschmolzen. In die Schmelze werden 10 Teile Glycerin, 1 Teil Ethylcellulose und 5 Teile Hopfenextrakt langsam eingerührt bis eine gleichmäßige Verteilung erreicht ist (Schmelze A).

18 Teile Gelatine, 10 Teile Polyvinylpyrrolidon, 5 Teile Natriumcarboximethylstärke und 11 Teile Saccharose werden bei Raumtemperatur mit 20 Teilen Wasser angeteigt, intensiv homogenisiert bis zur Erreichung einer gleichmäßigen Verteilung und anschließend unter Rühren auf 90°C erhitzt (Schmelze B).

Schmelze B wird bei 90°C langsam in Schmelze A eingerührt bis eine gleichmäßige Verteilung erreicht ist. Der Haftschmelzkleber wird dann mittels Rakelauftrag mit einem Flächengewicht von 300 g/m² auf einen silikonisierten Papierverbund gestrichen und vorübergehend mit einer silikonisierten HDPE-Folie abgedeckt.

Separat wird entsprechend obiger Formulierung ein zweiter hydrophiler Haftschmelzkleber hergestellt, der anstelle des Hopfenextrakts 5 Teile Baldrianextrakt enthält. Nach dem Ausstreichen auf einen silikonisierten Papierverbund und dem Erkalten wird dieser hydrophile Haftschmelzkleber auf den Hopfenextrakt-Haftschmelzkleber, von dem zuvor die HDPE-Zwischenabdeckung abgezogen wird, laminiert. Nach dem Abziehen des silikonisierten Papierverbundes von der Baldrianextraktschicht wird auf diese Schicht eine Polyurethanschicht kaschiert.

Aus dem Mehrschichtverbund werden Vorrichtungen mit einer Fläche von 30 cm² ausgestanzt, die in dem der Schutzschicht zugewandten Haftschmelzkleber 45 zug Hopfanextrakt und dem der Rückschicht zugewandten Haftschmelzkleber 45 mg Baldrianextrakt enthalten.

Die Vorrichtungen werden bei Applikation auf die Haut verwendet zur Erzielung einer beruhigenden Wirkung in beispielsweise Streßsituationen durch transdermale Abgabe der sedierend wirkenden Inhaltsstoffe des Baldrians und des Hopfens.

### Beispiel 17 :

24 Teile Pantothenylalkohol werden bei 90°C geschmolzen. In die Schmelze werden 10 Teile Glycerin und 2 Teile Ethylcellulose langsam eingerührt bis eine gleichmäßige Verteilung erreicht ist (Schmelze A).

18 Teile Natriumalginat, 10 Teile Polyvinylpyrrolidon, 5 Teile Natriumcarboximethylstärke und 11 Teile Saccharose werden bei Raumtemperatur mit 20 Teilen Wasser angeteigt, intensiv homogenisiert bis zur Erreichung einer gleichmäßigen Verteilung und anschließend unter Rühren auf 90°C erhitzt (Schmelze B).

Schmelze B wird bei 90°C langsam in Schmelze A eingerührt, bis eine gleichmäßige Verteilung erreicht ist. Der Haftschmelzkleber wird dann mittels Rakelauftrag mit einem Flächengewicht von 200 g/m² auf einen silikonisierten Papierverbund gestrichen; nach dem Erkalten wird eine silikonisierte HDPE-Zwischenabdeckung auflaminiert. Der Verbund wird so aufgeschnitten, daß Schmalrollen von 13 cm Breite erhalten werden.

Von einer solchen Schmalrolle wird die Zwischenabdeckung abgezogen, und es wird jeweils auf einer Fläche von 6 x 13 cm punktförmig eine Mischung enthaltend 1 ml Eukalyptusöl und 1 ml Kiefernnadelöl auf den Haftschmelzkleber aufgetragen. Auf diese Schicht wird, nach dem Abziehen der Zwischenabdeckung ein zweiter, 13 cm breiter hydrophiler Haftschmelzkleber kaschiert. Nach dem Abziehen einer der silikonisierten Papier-Schutzschichten wird eine mikroporöse Polyester-Rückschicht zukaschiert.

Es werden Vorrichtungen mit den Abmessungen 6 x 13 cm ausgestanzt und dicht eingesiegelt. Nach der gleichmäßigen Verteilung der ätherischen Öle im Haftschmelzkleber durch Diffusion kann die Vorrichtung bei Erkältungskrankheiten beispielsweise so verwendet werden, daß sie im oberen Brustbereich auf die Haut geklebt wird. Bei Erwärmen der Vorrichtung auf Körpertemperatur können die ätherischen Öle, die über die mikroporöse Rückschicht abgegeben und dann eingeatmet werden, ihre beschwerdelindernde Wirkung entfalten.

## Patentansprüche

1. Selbstklebendes Produkt zur Anwendung auf intakter Haut mit einem Haftkleber zu Abgabe von Stoffen an bzw. durch die Haut, die im Haftkleber gleichmäßig oder ungleichmäßig verteilt sind, dadurch gekennzeichnet, daß der Haftkleber ein durch Kombination von wasserlöslichen oder zumindest wasserquellbaren Polymeren mit wasserlöslichen, schmelzbaren Klebharzen gebildeter druckempfindlicher hydrophiler Haftschmelzkleber ist.

2. Produkt nach Anspruch 1, dadurch gekennzeichnet, daß der Haftschmelzkleber wasserlösliche, niedrigviskose, fädenziehende, klebrige Substanzen in Kombination mit hydrophilen Polymeren aufweist, die zusammenhängende Schichten von ausgezeichneter Kohäsion bilden und mit herkömmlichen Klebern vergleichbare Klebkräfte besitzt.

3. Produkt nach Anspruch 1 oder 2, dadurch gekennzeichnet, daß der hydrophile Haftschmelzkleber mindestens ein wasserlösliches, schmelzbares Klebharz und mindestens ein zumindest teilweise darin lösliches wasserlösliches oder zumindest wasserquellbares Polymer sowie abzugebenden Stoff aufweist.

4. Produkt nach einem der Ansprüche 1 bis 3, dadurch gekennzeichnet, daß der hydrophile Haftschmelzkleber eine Verarbeitungstemperatur zwischen 40 und 150°C, vorzugsweise zwischen 80 und 120°C hat.

5. Produkt nach einem der Ansprüche 1 bis 4, dadurch gekennzeichnet, daß der hydrophile Haftschmelzkleber als wasserlösliches, schmelzbares Klebharz Pantothenylalkohol, Honig, niedermolekulare Zucker wie z.B. Saccharose, Glucose und Fructose oder Derivate niedermolekularer Zucker wie beispielsweise Saccharoseacetatisobutyrat, sowie deren Kombinationen enthält.

6. Produkt nach einem der Ansprüche 1 bis 5, dadurch gekennzeichnet, daß der hydrophile Haftschmelzkleber als wasserlösliche oder zumindest wasserquellbare Polymere, Gelatine, Natriumalginat, Pektin, Carrageenan, Xanthan, Cellulosederivate, Stärke und Stärkederivate, Galaktomannan und Galaktomannanderivate, Polyvinylalkohol, Polyvinylpyrrolidon, Vinylpyrrolidon-Vinylacetat-Mischpolymerisate, Polyethylenglykole oder Polypropylenglykole oder deren Kombinationen enthält.

7. Produkt nach einem der Ansprüche 1 bis 6, dadurch gekennzeichnet, daß der hydrophile Haftschmelzkleber als abzugebenden Stoff Acetylsalicylsäure enthält.

8. Produkt nach einem der Ansprüche 1 bis 6, dadurch gekennzeichnet, daß der hydrophile Haftschmelzkleber als abzugebenden Stoff ein Steroidhormon, vorzugsweise Estradiol, enthält.

9. Produkt nach einem der Ansprüche 1 bis 6, dadurch gekennzeichnet, daß der hydrophile Haftschmelzkleber als abzugebenden Stoff ein eutektisches Gemisch von Lidocain und Prilocain enthält.

10. Produkt nach einem der Ansprüche 1 bis 6, dadurch gekennzeichnet, daß der hydrophile Haftschmelzkleber als abzugebenden Stoff eine oder mehrere pflanzliche Zubereitungen, wie beispielsweise Extrakte oder Tinkturen, enthält.

11. Produkt nach einem der Ansprüche 1 bis 6, dadurch gekennzeichnet, daß der hydrophile Haftschmelzkleber zerkleinerte Pflanzenteile, vorzugsweise Tabakpulver enthält.

12. Produkt nach einem oder mehreren der vorgenannten Ansprüche, dadurch gekennzeichnet, daß der hydrophile Haftschmelzkleber 0,1 bis 40% Wasser enthält.

13. Produkt nach einem oder mehreren der vorgenannten Ansprüche, dadurch gekennzeichnet, daß der hydrophile Haftschmelzkleber eine lipophile Phase enthält.

14. Produkt nach einem oder mehreren der vorgenannten Ansprüche, dadurch gekennzeichnet, daß der hydrophile Haftschmelzkleber Hilfsstoffe wie Feuchthaltemittel, Weichmacher, Penetrationsbeschleuniger, Konservierungsmittel, Desinfektionsmittel, pH-Regulatoren, Antioxidantien, Wirkstoffstabilisatoren, Emulsionsstabilisatoren, Duftstoffe, Farbstoffe, inerte Füllstoffe, Öle, Fette und/oder Wachse enthält.

15. Produkt nach einem oder mehreren der vorgenannten Ansprüche, dadurch gekennzeichnet, daß es eine flexible Rückschicht, einen damit verbundenen stoffenthaltenden hydrophilen Haftschmelzkleber, bei Abwesenheit anderer Steuermechanismen eine die Abgabe des Stoffs steuernde Membran, und fallweise eine mit der Membran verbundene Haftklebeeinrichtung zur Befestigung der Vorrichtung auf der Haut und eine die der Haut zugewandte, klebende Fläche der Vorrichtung abdeckende, vor der Applikation der Vorrichtung ablösbare Schutzschicht umfaßt.

16. Produkt nach einem oder mehren der vorgenannten Ansprüche, dadurch gekennzeichnet, daß es eine für leichtflüchtige Stoffe durchlässige Rückschicht umfasst.

17. Produkt nach einem oder mehreren der vorgenannten Ansprüche, dadurch gekennzeichnet, daß es einen hydrophilen Haftschmelzkleber mit poröser Struktur umfasst.

18. Produkt nach einem oder mehreren der vorgenannten Ansprüche, dadurch gekennzeichnet, daß sein stoffabgebender Teil mehrschichtig oder mehrteilig auf gebaut ist, wobei die einzelnen Schichten oder Teile unterschiedlich zusammengesetzt sein können.

19. Produkt nach einem oder mehreren der vorgenannten Ansprüche, dadurch gekennzeichnet, daß es mindestens teilweise biologisch abbaubar ist.

20. Verfahren zur Herstellung eines Produkts nach einem oder mehreren der vorgenannten Ansprüche, dadurch gekennzeichnet, daß die Bildung der Bestandteile des Prodükts, die hydrophile Haftschmelzkleber aufweisen, durch Extrusion, Gießen, Walzenauftrag, Rakelauftrag, Aufsprühen oder ein Druckverfahren erfolgt.

21. Verwendung des Produkts nach einem oder mehreren der Ansprüche 1 bis 19 bei der Herstellüng von Produkten der Human- und Veterinärmedizin oder Kosmetik.

22. Verwendung des Produkts nach einem oder mehreren der Ansprüche 1 bis 19 an Pflanzen.

## Claims

1. Self-adhesive product for application on intact skin, comprising a pressure-sensitive adhesive for the release of substances to or through the skin, said substances being distributed uniformly or non-uniformly in said pressure-sensitive adhesive, characterized in that the pressure-sensitive adhesive is a hydrophilic pressure-sensitive hot-melt adhesive formed by combining water-soluble or at least water-swellable polymers with water-soluble, meltable adhesive resins.

2. The product according to claim 1 characterized in that the pressure sensitive hot melt adhesive comprises water-soluble, ropy, low-viscous tacky substances in combination with hydrophilic polymers which form coherent layers of excellent cohesion and have adhesive forces comparable to those of conventional adhesives.

3. The product according to Claim 1 or 2, characterized in that the hydrophilic pressure-sensitive hot-melt adhesive comprises at least one water-soluble, meltable adhesive resin and at least one polymer which is at least partially soluble therein or is at least water-swellable.

4. The product according to claims 1 to 3 characterized in that the hydrophilic pressure sensitive hot-melt adhesive has a processing temperature between 40 and 150°C, preferably between 80 and 120°C.

5. The product according to claims 1 to 4 characterized in that the hydrophilic pressure sensitive hot-melt adhesive comprises pantothenyl alcohol, honey, low-molecular sugars, such as saccharose, glucose, and fructose, or derivatives of low-molecular sugars, such as saccharose acetate isobutyrate, as well as their combinations, as water-soluble, meltable adhesive resin.

6. The product according to claims 1 to 5 characterized in that the hydrophilic pressure sensitive hot-melt adhesive preferably comprises gelatin, sodium alginate, pectin, carrageenan, xanthan, cellulose derivatives, starch and starch derivatives, galactomannan and galactomannan derivatives, polyvinyl alcohol, polyvinyl-pyrrolidone, vinyl-pyrrolidone-vinyl-acetate copolymers, polyethylene glycols, or polypropylene glycols, or their combinations, as water-soluble, or at least water-swellable, polymers.

7. The product according to claims 1 to 6 characterized in that the hydrophilic pressure sensitive hot-melt adhesive comprises actetylsalicylic acid as substance to be released.

8. The product according to claims 1 to 6 characterized in that the hydrophilic pressure sensitive hot-melt adhesive comprises a steroid hormone, preferably estradiol, as substance to be released.

9. The product according to claims 1 to 6 characterized in that the hydrophilic pressure sensitive hot-melt adhesive comprises a eutectic mixture of lidocaine and prilocaine as substance to be released.

10. The product according to claims 1 to 6 characterized in that the hydrophilic pressure sensitive hot-melt adhesive comprises one or several vegetable preparations, for example, extracts or tinctures, as substance to be released.

11. The product according to claims 1 to 6 characterized in that the hydrophilic pressure sensitive hot-melt adhesive comprises triturated plant portions, preferably tobacco powder.

12. The product according to one or several of the preceding claims characterized in that the hydrophilic pressure sensitive hot-melt adhesive comprises 0.1 to 40% of water.

13. The product according to one or several of the preceding claims characterized in that the hydrophilic pressure sensitive hot-melt adhesive comprises a lipophilic phase.

14. The product according to one or several of the preceding claims characterized in that the hydrophilic pressure sensitive hot-melt adhesive comprises adjuvants, such as humectants, softening agents, penetration enhancers, preservatives, disinfectants, pH-regulators, antioxidants, active substance stabilizers, emulsion stabilizers, odorous substances, dyes, inert fillers, oils, fats, and/or waxes.

15. The product according to one or several of the preceding claims characterized in that the product comprises a flexible backing layer, an active substance-containing, hydrophilic pressure sensitive hot-melt adhesive connected thereto, in the absence of other controlling mechanisms a membrane controlling the release of the substances, and, if necessary, a pressure sensitive adhesive means connected to the membrane for the fixation of the product to the skin, and a protective layer which covers an adhesive surface of the product facing the skin and which is removed prior to the application of the product.

16. The product according to one or several of the preceding claims characterized in that the product comprises a backing layer which is permeable to highly volatile substances.

17. The product according to one or several of the preceding claims characterized in that the product comprises a hydrophilic pressure sensitive hot-melt adhesive having a porous structure.

18. The product according to one or several of the preceding claims characterized in that the substance-releasing portion of the product has a multi-layer or multi-part structure wherein the individual layers or parts may have a different composition.

19. The product according to one or several of the preceding claims characterized in that the product is at least partially biodegradable.

20. A process for the production of a product according to one or several of the preceding claims, characterized in that the formation of the components of the product, which have hydrophilic pressure sensitive hot-melt adhesives, is effected by extrusion, casting, roll coating, knife coating, spraying, or by a printing process.

21. The use of the product according to one or several of Claims 1 to 19 in the production of products for human or veterinary medicine or for cosmetics.

22. The use of the product according to one or several of Claims 1 to 19 in plants.

## Revendications

1. Produit auto-adhésif à des fins d'application sur une peau intacte comprenant une colle à fusion pour le dégagement de substances, sur respectivement à travers la peau, qui sont distribuées de manière régulière ou de manière irrégulière dans la colle à fusion, caractérisé en ce que la colle à fusion est une colle de contact thermofusible, hydrophile, sensible à la pression, obtenue par combinaison de polymères hydrosolubles ou au moins aptes à gonfler dans l'eau avec des résines thermofusibles hydrosolubles.

2. Produit selon la revendication 1, caractérisé en ce que la colle de contact thermofusible présente des substances collantes, filantes, faiblement visqueuses, hydrosolubles en combinaison avec des polymères hydrophiles, qui forment des couches cohésives manifestant une cohésivité excellente, et possède des forces d'adhérence comparables à celles de colles habituelles.

3. Produit selon la revendication 1 ou 2, caractérisé en ce que la colle de contact thermofusible hydrophile présente au moins une résine thermofusible hydrosoluble et au moins un polymère hydrosoluble ou au moins apte à gonfler dans l'eau, au moins en partie soluble dans la colle thermofusible de contact, ainsi qu'une substance à dégager.

4. Produit selon l'une quelconque des revendications 1 à 3, caractérisé en ce que la colle de contact thermofusible hydrophile possède une température de traitement entre 40 et 150°C, de préférence, entre 80 et 120°C.

5. Produit selon l'une quelconque des revendications 1 à 4, caractérisé en ce que la colle de contact thermofusible hydrophile contient, à titre de résine thermofusible hydrosoluble, de l'alcool pantathénylique, du miel, des sucres à bas poids moléculaire tels que par exemple le saccharose, le glucose, et le fructose ou encore des dérivés de sucres à bas poids moléculaire, tels que par exemple l'acéto-isobutyrate de saccharose, ainsi que leurs mélanges.

6. Produit selon l'une quelconque des revendications 1 à 5, caractérisé en ce que la colle de contact thermofusible hydrophile contient, à titre de polymères hydrosolubles ou au moins aptes à gonfler dans l'eau, de la gélatine, de l'alginate de sodium, de la pectine, de la carragheenine, du xanthane, des dérivés de la cellulose, de l'amidon et des dérivés de l'amidon, du galactomannane et des dérivés du galactomannane, de l'alcool polyvinylique, de la polyvinylpyrrolidone, des copolymères de vinylpyrrolidone-acétate de vinyle, des polyéthylèneglycols ou des polypropylèneglycols ou encore leurs mélanges.

7. Produit selon l'une quelconque des revendications 1 à 6, caractérisé en ce que la colle de contact thermofusible hydrophile contient, à titre de substance à dégager, de l'acide acétylsalicylique.

8. Produit selon l'une quelconque des revendications 1 à 6, caractérisé en ce que la colle de contact thermofusible hydrophile contient, à titre de substance à dégager, une hormone stéroïdienne, de préférence l'oestradiol.

9. Produit selon l'une quelconque des revendications 1 à 6, caractérisé en ce que la colle de contact thermofusible hydrophile contient, à titre de substance à dégager, un mélange eutectique de lidocaïne et de prilocaïne.

10. Produit selon l'une quelconque des revendications 1 à 6, caractérisé en ce que la colle de contact thermofusible hydrophile contient, à titre de substance à dégager, une ou plusieurs préparations végétales telles que par exemple des extraits ou des teintures.

11. Produit selon l'une quelconque des revendications 1 à 6, caractérisé en ce que la colle de contact thermofusible hydrophile contient des extraits de plantes broyées, de préférence de la poudre de tabac.

12. Produit selon une ou plusieurs des revendications précédentes, caractérisé en ce que la colle de contact thermofusible hydrophile contient de l'eau à concurrence de 0,1 à 40%.

13. Produit selon une ou plusieurs des revendications précédentes, caractérisé en ce que la colle de contact thermofusible hydrophile contient une phase lipophile.

14. Produit selon une ou plusieurs des revendications précédentes, caractérisé en ce que la colle de contact thermofusible hydrophile contient des adjuvants tels que des agents qui maintiennent l'humidité, des plastifiants, des accélérateurs de la pénétration, des conservants, des désinfectants, des régulateurs du pH, des antioxydants, des stabilisateurs du principe actif, des stabilisateurs d'émulsion, des substances odorantes, des colorants, des matières de charge inertes, des huiles, des graisses et/ou des cires.

15. Produit selon une ou plusieurs des revendications précédentes, caractérisé en ce qu'il comprend une couche dorsale flexible; une colle de contact thermofusible hydrophile contenant des substances liée à la première citée; en l'absence d'autres mécanismes de commande, une membrane qui commande le dégagement de la substance; et le cas échéant, un mécanisme de collage par contact lié à la membrane, pour fixer le dispositif sur la peau; et une couche de protection pelliculable avant l'application du dispositif, recouvrant la surface collante du dispositif tournée vers la peau.

16. Produit selon une ou plusieurs des revendications précédentes, caractérisé en ce qu'il comprend une couche dorsale perméable pour des substances aisément volatiles.

17. Produit selon une ou plusieurs des revendications précédentes, caractérisé en ce qu'il comprend une colle de contact thermofusible hydrophile de structure poreuse.

18. Produit selon une ou plusieurs des revendications précédentes, caractérisé en ce que sa partie dégageant la substance est constituée de plusieurs couches ou de plusieurs parties, les couches ou les parties individuelles pouvant être de composition différente.

19. Produit selon une ou plusieurs des revendications précédentes, caractérisé en ce qu'il est au moins en partie biodégradable.

20. Procédé pour la préparation d'un produit selon une ou plusieurs des revendications précédentes, caractérisé en ce que la formation des constituants du produit qui présentent des colles de contact thermofusibles a lieu par extrusion, par coulage, par application au rouleau, par application à la racle, par pulvérisation ou à l'aide d'un procédé d'impression.

21. Utilisation du produit selon une ou plusieurs des revendications 1 à 19 lors de la préparation de produits de la médecine humaine et vétérinaire ou de la cosmétique.

22. Utilisation du produit selon l'une ou plusieurs des revendications 1 à 19 sur des plantes.
